# EUROPÄISCHE PATENTANMELDUNG

(11) **EP 2 586 767 A1**
(43) Veröffentlichungstag der Anmeldung: **01.05.2013**
(21) Anmeldenummer: 11186487.2
(22) Anmeldetag: 25.10.2011
(51) Int. Cl.: C07C 68/02

(54) **Verfahren zur Herstellung von Diarylcarbonaten und Polycarbonaten**

(71) Anmelder: Bayer MaterialScience AG, 51373 Leverkusen (DE)
(72) Erfinder: Ooms, Pieter Dr., 47800 Krefeld (DE)
(74) Vertreter: BIP Patents

(57) **Zusammenfassung**

Die vorliegende Erfindung betrifft ein Verfahren zur kontinuierlichen Herstellung von Diarylcarbonaten aus Phosgen und wenigstens einer Monohydroxyverbindung (Monophenol) in Gegenwart von Katalysa-toren, sowie deren Verwendung zur Herstellung von Polycarbonaten. Der dabei anfallende Chlor-wasserstoff wird durch elektrochemische oder thermische Oxidation zu Chlor umgesetzt, wobei das Chlor zur Herstellung des Phosgens rückgeführt wird. Insbesondere besteht das Verfahren in der Wiederverwendung des Katalysators für das Diphenylcarbonat-Herstellungsverfahren (DPC-Verfahren).

## Beschreibung

Die vorliegende Erfindung betrifft ein Verfahren zur kontinuierlichen Herstellung von Diarylcarbonaten aus Phosgen und wenigstens einer Monohydroxyverbindung (Monophenol) in Gegenwart von Metall enthaltenden Katalysatoren, sowie deren Verwendung zur Herstellung von Polycarbonaten. Der dabei anfallende Chlorwasserstoff wird durch elektrochemische oder thermische Oxidation zu Chlor umgesetzt, wobei das Chlor zur Herstellung des Phosgens rückgeführt wird. Insbesondere besteht das Verfahren in der Wiederverwendung des Metall enthaltenden Katalysators für das Diphenylcarbonat-Herstellungsverfahren (DPC-Verfahren).

Es ist bekannt, dass Diarylcarbonate, insbesondere Diphenylcarbonat durch Phasengrenzflächenphosgenierung (Schotten-Baumann-Reaktion) von Monophenolen in einem inerten Lösungsmittel in Gegenwart von Alkali und einem Katalysator hergestellt werden können. Dabei wirkt sich die Verwendung von Lösungsmitteln und Natronlauge nachteilig aus, da durch die wässrige Lauge eine teilweise Verseifüng von Phosgen oder Chlorkohlensäureester stattfinden kann, große Mengen Kochsalz als Nebenprodukt anfallen und das Lösungsmittel und Katalysator zurückgewonnen werden müssen.

Aus dem Grund ist auch die Herstellung von Diarylcarbonaten und insbesondere Diphenylcarbonat durch Reaktion von Monophenolen und Phosgen ohne Alkali und ohne Verwendung von Lösungsmitteln in Gegenwart von einem Katalysator durch den Direktphosgenierungsprozess untersucht worden und prinzipiell in der Literatur beschrieben.

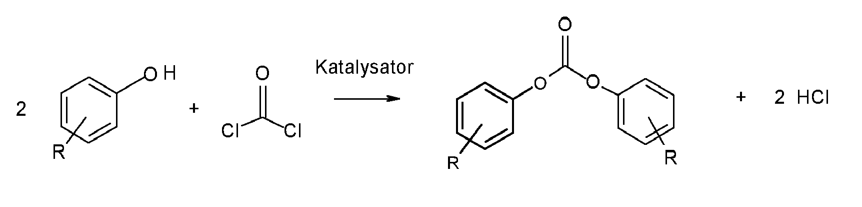

Vorschläge für Verfahren ohne Lösungsmittel mit löslichen Katalysatoren werden in US 2 837 555, 3 234 263 und 2 362 865 beschrieben.

Auch zu der Verwendung von heterogenen, nicht löslichen Katalysatoren, die eine Aufarbeitung des Reaktionsgemisches wesentlich erleichtern, wurden Vorschläge gemacht. So wird in der EP 516 355 A2 vor allem Aluminiumtrifluorid empfohlen, das auf Träger wie Alumosilikate aufgebracht wird. Die Synthese von Aluminiumfluorid ist jedoch durch die Handhabung von Fluor oder Flußsäure sehr aufwendig und teurer.

Weiter werden in der WO 91/06526 Metallsalze auf porösen Trägern als Katalysatoren für die erfindungsgemäßen Umsetzungen beschrieben. Eine vollkontinuierliche Phosgenierung von Phenol an solchen Katalysatoren ist nur in der Gasphase möglich, was aber relativ hohe Reaktionstemperaturen und die Gefahr der Zersetzung der empfindlichen Chlorameisensäureester mit sich bringt. Offensichtlich ist eine Phosgenierung von Phenol mit diesen Katalysatoren in der Flüssigphase nicht durchführbar, da das heiße, flüssige Phenol die aktiven Katalysatorbestandteile auswäscht.

Deshalb wurden nicht-geträgerte Katalysatoren vorgeschlagen, die den großen Vorteil haben, dass man den Katalysator sehr leicht abtrennen kann und keine Verunreinigungen im rohen Reaktionsprodukt verbleiben. Die Aufarbeitung wird dadurch wesentlich vereinfacht.

So wurden Verfahren zur Herstellung von Diarylcarbonaten durch Phosgenierung von Monophenolen in Gegenwart von heterogenen Katalysatoren wie Aktivkohlen (EP 483 632), Aluminiumoxiden (EP 635 477), Alumosilikaten (EP 635 476), Metalloxiden (EP 645 364), Metallaten (EP 691 326), Hartstoffe (EP 722 930) und Mischhydroxiden (DE 10 2008 050 828) als auch homogene Katalysatoren wie Metallsalze (US 634622), aromatische Stickstoffheterocyclen (D-OS 2 447 348) und Organophosphorverbindungen (US-5 136 077) sowohl in der Flüssigphase (EP 757 029, EP 791 574) als auch in der Gasphase (EP 808 821) beschrieben.

Nach der Synthese des Diarylcarbonats wird das Diarylcarbonat als Mischung mit dem eingesetzten Monophenol und gegebenenfalls Katalysator oder gegebenenfalls in Form seiner Lösung in dem bei der Synthese verwendeten organischen Lösungsmittel, beispielsweise Chorbenzol, abgetrennt.

Zum Erhalt des hochreinen Diarylcarbonats kann eine Reinigung durch Destillation und / oder Kristallisation erfolgen. Beispielsweise erfolgt dies durch eine oder mehrere hintereinander geschaltete Destillationskolonnen bei der gegebenenfalls das Lösungsmittel vom Diarylcarbonat abgetrennt wird.

Diese Reinigungsstufe bzw. Stufen können beispielsweise kontinuierlich so geführt werden, dass die Sumpftemperatur bei der Destillation 150°C bis 310°C, bevorzugt 160 bis 230°C beträgt. Der zur Durchführung dieser Destillation angewendete Druck beträgt dabei insbesondere 1 bis 1000 mbar, bevorzugt 5 bis 100 mbar.

Die so gereinigten Diarylcarbonate zeichnen sich durch besonders hohe Reinheit (GC >99,95%) und sehr gutes Umesterungsverhalten aus, so dass hieraus anschließend ein Polycarbonat in exzellenter Qualität hergestellt werden kann.

### Neue Beschreibungsseite 3

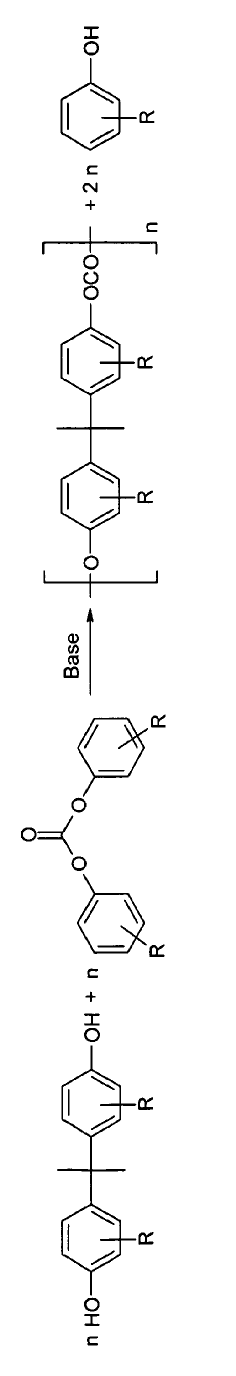

### Neue Beschreibungsseite 3a

Die Verwendung der Diarylcarbonate zur Herstellung von aromatischen Oligo- / Polycarbonaten nach dem Schmelzumesterungsverfahren ist literaturbekannt und beispielsweise in der Encyclo-pedia of Polymer Science, Vol. 10 (1969), Chemistry and Physics of Polycarbonates, Polymer Reviews, H. Schnell, Vol. 9, John Wiley and Sons, Inc. (1964), S. 50/51 oder der US-A-5 340 905 beschrieben.

Die Nutzung des bei der Herstellung von Diphenylcarbonat durch Direktphosgenierung von Phenol gebildeten Chlorwasserstoffs kann z.B. durch Vermarktung der wässrigen Lösung (Salzsäure) oder durch Verwendung bei Synthesen anderer chemischer Produkte erfolgen.

Eine der gängigsten Nutzungsmöglichkeiten für den anfallenden Chlorwasserstoff ist daher die Verwendung als Rohstoff bei der PVC-Herstellung, bei dem die Oxychlorierung von Ethylen mit Chlorwasserstoff zu Ethylendichlorid erfolgt.

Ein Recyclingverfahren für den Chlorwasserstoff und die Rückführung des Chlors in den Diphenylcarbonat-Produktionsprozess, in dem der Chlorwasserstoff anfällt, ist daher die angestrebte Verfahrensweise.

Eine Übersicht über elektrochemische Recycling-Verfahren wird in dem Artikel "Chlorine Regeneration from Anhydrous Hydrogen Chloride" von Dennie Turin Mah, veröffentlicht in "12th International Forum Electrolysis in Chemical Industry - Clean and Efficient Processing Electrochemical Technoogy for Synthesis, Separation, Recycle and Environmental Improvement, October 11-15, 1998, Sheraton Sand Key, Clearwater Beach, FL", gegeben.

Die Rückführung von Chlorwasserstoff durch elektrochemische Oxidation unter Bildung von Chlor und Wasserstoff ist in LU 88 569 und EP 1 112 997 beschrieben. Nachteilig ist die geringe Stromausbeute und die Produktion von Wasserstoff, der für das Polycarbonatherstellungsprozess keine Verwendung hat.

Die Rückführung von Chlorwasserstoff durch elektrochemische Oxidation unter Verwendung einer Gasdiffusionselektrode als Kathode ist beispielsweise in EP 2 371 806 A1 beschrieben.

Die Rückführung von Chlorwasserstoff durch thermische Oxidation (Deacon-Verfahren) ist beispielsweise in EP 2 371 807 A1 beschrieben.

EP 1 016 648 A1 beschreibt eine Reinigung des Katalysators Titantetraphenolat, eingesetzt bei der phosgenfreien Herstellung von Diphenylcarbonat durch Umesterung von Dimethylcarbonat mit Phenol, durch Hydrolyse zu Titanoxid. Eine Wiederverwendung erfordert anschließend aufwendige Herstellungschritte zum Katalysator.

Ausgehend vom oben geschilderten Stand der Technik bestand die Aufgabe der vorliegenden Erfindung darin, ein Diarylcarbonat-Herstellungsverfahren bereitzustellen, welches Produkte in hoher Reinheit und guter Ausbeute liefert und eine Reduzierung der Umweltbelastung bzw. Abwasserproblematik in den Klärwerken durch maximierte Rückführung von Nebenprodukten, die aus der Polycarbonat-Produktion stammen, erreicht wird. Insbesondere sollte das Recycling mit minimalem Energieeinsatz und daher resourcenschonend erfolgen.

Es wurde nun gefunden, dass sich nach Destillation des Diphenylcarbonats der Katalysator-haltige Destillationsrückstand besonders günstig wiederverwerten lässt, wenn dieser-wieder als Katalysator bei der Direktphosgenierung von Phenol zur Herstellung von Diphenylcarbonat genutzt wird.

Der nach der kontinuierlichen Herstellung von Diarylcarbonaten durch Reaktion von Monophenolen und Phosgen in Gegenwart von Metall-Katalysatoren nach Destillation als schwerflüchtige Rückstand erhaltene Katalysatorfraktion kann ohne aufwendige Reinigung gegebenenfalls nach einfacher Behandlung mit Chlorierungsmitteln wie Phosgen, Chlorameisensäurephenylester oder Chlorwasserstoff wieder der Direktphosgenierung von Phenol zugeführt werden.

Der bei der kontinuierlichen Herstellung von Diarylcarbonaten durch Reaktion von Monophenolen und Phosgen in Gegenwart von Katalysatoren anfallende Chlorwasserstoff kann ohne aufwendige Reinigung gegebenenfalls nach einfacher Behandlung mit Aktivkohle direkt einer thermischen Oxidation oder als wässrige Lösung einer elektrochemischen Oxidation zu Chlor und Wasser bzw. Wasserstoff zugeführt werden, wobei das Chlor zur Herstellung des Phosgens rückgeführt werden kann.

Gegenstand der vorliegenden Erfindung ist demnach ein Verfahren zur Herstellung von Diarylcarbonaten ausgehend von Monophenolen und Carbonyldihalogenid in Gegenwart von Metall-Katalysatoren, das dadurch gekennzeichnet ist, dass der dabei eingesetzte Katalysator nach Abtrennung des Diarylcarbonats direkt oder nach Behandlung mit Chlorierungsmitteln wiederverwendet wird für das Diphenylcarbonat-Herstellungsverfahren (DPC-Verfahren).

Der entstehende Halogenwasserstoff wird durch Elektrolyse oder thermische Oxidation in das Halogen und dieses wiederum nach Isolierung und Reaktion mit Kohlenmonoxid zum Carbonyldihalogenid umgesetzt, welches anschließend wieder mit dem Monophenol zur Herstellung des Diarylcarbonats eingesetzt wird. Das Monophenol, das bei der Herstellung von lösungsmittelfreiem Polycarbonat durch Umesterung von Diarylcarbonaten und Bisphenolen freigesetzt wird, kann wiederum zur Herstellung des Diarylcarbonats verwendet werden. Weiterhin betrifft die Anmeldung ein Verfahren in dem das hergestellte Diarylcarbonat zur Herstellung von Polycarbonat verwendet wird.

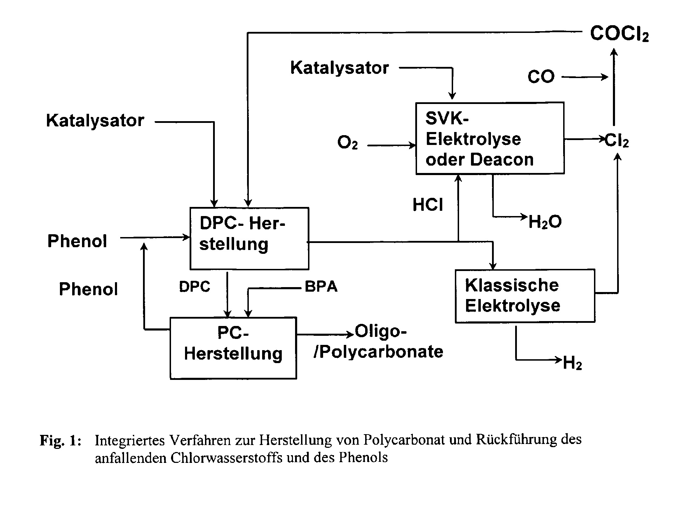

Das erfindungsgemäße Gesamtverfahren ist flexibel, einfach in der Durchführung und liefert Produkte in hoher Reinheit, die für den Gesamtprozess außerordentlich wichtig sind unter gleichzeitiger Reduzierung der Umweltbelastung durch Wiederverwendung, umfassend folgende Prozessschritte (vgl. Fig. 1):
(a) Herstellung von Phosgen durch Umsetzung von Chlor mit Kohlenmonoxid,
(b) Umsetzung des gemäß Schritt (a) gebildeten Phosgens mit wenigstens einem Monophenol in Gegenwart eines Metall-Katalysators, und gegebenenfalls organischem Lösungsmittel unter Bildung wenigstens eines Diarylcarbonats und von Chlorwasserstoff,
(c) Abtrennung und Aufarbeitung des in Schritt (b) gebildeten Diarylcarbonats
(d) Rückführung der gemäß Schritt (c) zurückbleibenden schwerflüchtigen Metall enthaltenden Katalysatorfraktion in die Herstellung des Diarylcarbonats gemäß Schritt (b),
(e) Abtrennung und gegebenenfalls Reinigung des gemäß Schritt (b) gebildeten Chlorwasserstoffs,
(f) elektrochemische oder thermische Oxidation wenigstens eines Teils des Chlorwasserstoffs aus (e) zu Chlor
(g) Rückführung wenigstens eines Teils des gemäß Schritt (f) hergestellten Chlors in die Herstellung von Phosgen gemäß Schritt (a),
wobei gegebenenfalls die Metall enthaltende Katalysatorfraktion vor der Rückführung mit Chlorierungsmitteln wie beispielsweise Phosgen, Chlorameisensäurephenylester oder Chlorwasserstoff umgesetzt und gereinigt wird.

Die Durchführung der Elektrolyse kann klassisch oder mit einer Gasdiffusionselektrode als Sauerstoffverzehtkathode durchgeführt werden.

Durch Behandlung mit Chlorierungsmitteln RCI wird der Metall-haltige Destillationsrück-stand in Metallchlorid und Monophenol überführt. Das gebildete Metallchlorid wird über-destilliert und dem Diarylcarbonatherstellungsverfahren wieder als Katalysator zugeführt, wobei insbesondere die schwerflüchtigere substituierte Aromate, wie beispielsweise Salicyl-säurephenylester, zurückbleiben.

M(OAr¹)ₓ (OAr²)_{y}Cl_{n-x-y} + (x+y) RCl → MClₙ+ x Ar¹OR + y Ar²OR

wobei
M für Ti, Zr oder Al,
n für eine Zahl 3 oder 4,
x für eine Zahl von 0 bis 4 ,
y für eine Zahl von 0 bis 4,
wobei x + y = n
R für ClCO, C₆H₅CO, H,
Ar¹ für Phenyl und
Ar² für ein Chlor, Alkyl, Aryl, Aryloxy, Alkyloxycarbonyl oder Aryloxycarbonyl substituierter Aromat, beispielsweise C₆H₄Cl, C₆H₄CH₃ oder C₆H₄COOC₆H₅ steht.

In einer besonders bevorzugten Ausführungsform wird wenigstens ein Teil des gemäß Schritt (c) hergestellten Diarylcarbonats mit einem Bisphenol zum Oligo-/Polycarbonat und dem Monophenol umgesetzt (die sogenannte Umesterungsreaktion). Das bei der Umesterung entstehende Monophenol kann in einer weiteren bevorzugten Ausführungsform wiederum in Schritt (b) eingesetzt werden.

Das erfindungsgemäße Verfahren ist ein Verfahren zur Herstellung von Diarylcarbonaten unter Rückführung der dabei eingesetzte Katalysator nach Abtrennung des Diarylcarbonats direkt oder nach Behandlung mit Chlorierungsmitteln.

Im ersten Schritt (a) des erfindungsgemäßen Verfahrens erfolgt die Herstellung von Phosgen durch Umsetzung von Chlor mit Kohlenmonoxid. Die Synthese von Phosgen ist hinlänglich bekannt und ist z.B. in Ullmanns Enzyklopädie der industriellen Chemie, 3. Auflage, Band 13, Seite 494-500 dargestellt. Im technischen Maßstab wird Phosgen überwiegend durch Umsetzung von Kohlenmonoxid mit Chlor bevorzugt an Aktivkohle als Katalysator hergestellt. Die stark exotherme Gasphasenreaktion erfolgt bei Temperaturen von mindestens 250°C bis maximal 600°C in der Regel in Rohrbündelreaktoren. Die Abführung der Reaktionswärme kann auf unterschiedliche Weise erfolgen, beispielsweise durch ein flüssiges Wärmetauschmittel, wie z.B. in WO 03/072237 beschrieben, oder durch Siedekühlung über einen Sekundärkühlkreislauf unter gleichzeitiger Nutzung der Reaktionswärme zur Dampferzeugung, wie z.B. in US 4 764 308 offenbart.

Aus dem gemäß Schritt (a) gebildeten Phosgen wird durch Umsetzung mit wenigstens einem Monophenol in einem nächsten Verfahrensschritt (b) wenigstens ein Diarylcarbonat gebildet. Der Verfahrensschritt (b) wird nachfolgend auch als Phosgenierung bezeichnet. Die Umsetzung erfolgt unter Bildung von Chlorwasserstoff als Nebenprodukt.

Die Synthese von Diarylcarbonaten ist ebenfalls aus dem Stand der Technik hinlänglich bekannt, wobei in der Regel das Monophenol in einem stöchiometrischen Überschuss, bezogen auf das Phosgen, eingesetzt wird. Üblicherweise findet die Phosgenierung gemäß (b) in der Flüssigphase statt, wobei das Phosgen und das Monophenol in der Schmelze, gegebenenfalls in einem Lösungsmittel gelöst sein können. Bevorzugte Lösungsmittel sind chlorierte aromatische Kohlenwasserstoffe, wie beispielsweise Chlorbenzol, o-Dichlorbenzol, p-Dichlorbenzol, Trichlorbenzole, die entsprechenden Chlortoluole oder Chlorxylole, Toluol, Xylole und das Monophenol selbst.

Besonders bevorzugt ist das geschmolzene Monophenol als Lösungsmittel.

In einer anderen, bevorzugten Ausführungsform findet die Phosgenierung in der Gasphase statt. Die Gasphasenphosgenierung ist z.B. in US 5 831 111 beschrieben.

Für das erfindungsgemäße Verfahren geeignete Monophenole sind Phenole der Formel (I) 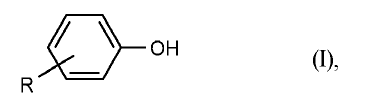 worin
- R: Wasserstoff, Halogen oder ein verzweigter oder unverzweigter C₁- bis C₉-Alkylrest, C₁- bis C₉-Alkoxyrest oder C₁ bis C₉-Alkoxycarbonylrest ist.

Bevorzugt sind also Phenol, Alkylphenole wie Kresole, p-tert.-Butylphenol, p-Cumylphenol, p-n-Octylphenol, p-iso-Octylphenol, p-n-Nonylphenol und p-iso-Nonylphenol, Halogenphenole wie p-Chlorphenol, 2,4-Dichlorphenol, p-Bromphenol, 2,4,6-Tribromphenol, Anisol und Salicylsäuremethyl oder -phenylester. Besonders bevorzugt ist Phenol.

Für das erfindungsgemäße Verfahren können heterogene als auch homogene Katalysatoren eingesetzt werden.

Als heterogene Katalysatoren können Aktivkohlen (EP 483 632), Aluminiumoxide (EP 635 477), Alumosilikate (EP 635 476), Metalloxide (EP 645 364), Metallate (EP 691 326) Hartstoffe (EP 722 930) und Mischhydroxide (DE 10 2008 050 828) sowohl in der Flüssigphase (EP 757 029, EP 791 574) als auch in der Gasphase (EP 808 821) eingesetzt werden.

Als homogene Katalysatoren können Metallverbindungen wie Metallsalze, -halogenide, -alkylate und -phenolate, aromatische Stickstoffheterocyclen oder Organophosphorverbindungen eingesetzt werden.

Es können ein oder mehrere, aktivierte oder nicht-aktivierte Katalysatoren eingesetzt werden.

Geignete Katalysatoren für Schritt b) des erfindungsgemäßen Verfahren sind:
Metallsalze, -halogenide, -alkylate und -phenolate wie z.B. AlCl₃, Al(OC₆H₅)₃, AlF₃, ZrCl₄, Zr(OC₆H₅)₄, TiCl₄ oder Ti(OC₆H₅)₄,

Bevorzugt sind-Titanchlorid, Zirkonchlorid, Aluminiumchlorid, Titanphenolat, Zirkonphenolat und Aluminiumphenolat, die als homogene Katalysatoren eingesetzt werden.

Besonders bevorzugt sind TiCl₄, Aluminiumchlorid Zirkonchlorid und Titanphenolat.

Der Katalysator wird in Mengen von 0,5 bis 100 Gew.-% bezogen auf die Menge an Monohydroxyverbindung (Monophenol), bei nicht vollkontinuierlicher Fahrweise bzw. mit Belastungen von 0,1 bis 20 g Monohydroxyverbindung (Monophenol) pro g Katalysator pro Stunde bei vollkontinuierlicher Fahrweise, einge-setzt.

Phosgen kann in dem Verfahrensschritt b) flüssig, gasförmig oder gegebenenfalls gelöst in einem inerten Lösungsmittel eingesetzt werden.

In dem neuen Verfahren in Schritt b) gegebenenfalls verwendbare inerte organische Lösungsmittel sind beispielsweise Toluol, Chlorbenzol, Dichlorbenzol und Chlortoluol, bevorzugt ist Phenol selbst.

Wird ein Phosgenierung in der Gasphase gewählt, so erfolgt die Umsetzung oberhalb der Siedetemperatur des Phenols, bevorzugt innerhalb einer mittleren Kontaktzeit von 0,5 bis 5 Sekunden und bei Temperaturen von 180 bis 500°C.

Bei der Phosgenierung in der Flüssigphase werden üblicherweise Temperaturen von 20 bis 240°C und Drücke von 1 bis ca 50 bar eingesetzt. Die Phosgenierung in der Flüssigphase kann einstufig oder mehrstufig durchgeführt werden, wobei im allgemeinem Phenol im stöchiometrischen Überschuss eingesetzt werden kann.

Dabei können das Phenol und das Phosgen über ein statisches Mischelement vereinigt, in Gegen-oder Gleichstrom beispielsweise über ein Festbett (heterogen) oder in eine oder mehrere Reaktivdestillationskolonnen oder Blasensäulen (homogen) geführt werden, wo das Gemisch zum gewünschten Diarylcarbonat und Chlorwasserstoff abreagiert. Neben Reaktionstürmen, die mit geeigneten Mischelementen versehen sind, können auch Reaktionsbehälter mit Rührvorrichtung eingesetzt werden. Außer statischen Mischelementen können auch spezielle dynamische Mischelemente Anwendung finden. Geeignete statische und dynamische Mischelemente sind aus dem Stand der Technik bekannt.

Nach der Phosgenierung gemäß Schritt (b) erfolgt erfindungsgemäß in Schritt (c) die Abtrennung der bei der Phosgenierung gebildeten Diarylcarbonate. Dies geschieht dadurch, dass zunächst das Reaktionsgemisch der Phosgenierung in einen flüssigen und einen gasförmigen Produktstrom in einer dem Fachmann bekannten Weise aufgetrennt wird. Der flüssige Produktstrom enthält im Wesentlichen das Diarylcarbonat, den metall-enthaltenden Katalysator, gegebenenfalls das Lösungsmittel sowie einen geringen Teil an nicht umgesetztem Phosgen. Der gasförmige Produktstrom besteht im Wesentlichen aus Chlorwasserstoffgas, stöchiometrisch überschüssigem Phosgen, sowie geringfügigen Mengen an gegebenenfalls Lösungsmittel und Inertgasen, wie zum Beispiel Stickstoff und Kohlenmonoxid. Ferner wird der Flüssigstrom gemäß Schritt (c) anschließend einer Aufarbeitung zugeführt, vorzugsweise einer destillativen Aufarbeitung, wobei nacheinander Phosgen sowie gegebenenfalls das Lösungsmittel abgetrennt werden. Gegebenenfalls erfolgt gemäß Schritt (c) außerdem eine weitere Aufarbeitung der gebildeten Diarylcarbonate. Dies geschieht beispielsweise, indem das erhaltene Diarylcarbonat in einer dem Fachmann bekannten Weise gereinigt wird durch Destillation oder Kristallisation.

Die Reinigung des anfallenden Chlorwasserstoffs ist in EP 2 371 806 A1 für die Rückführung durch elektrochemische Oxidation und in EP 2 371 807 A1 für die Rückführung durch ther-mische Oxidation (Deacon-Verfahren) beschrieben.

Erfindungsgemäß wird im nächsten Verfahrensschritt (g) wenigstens ein Teil des gemäß Schritt (f) hergestellten Chlors in die Herstellung von Phosgen gemäß Schritt (a) rückgeführt.

Vor der Rückführung wird das Chlor vorzugsweise in einer ein- oder mehrstufigen Kühlung mittels eines Kühlaggregats, z.B. eines Röhrenwärmetauscher, abgekühlt und getrocknet. Die Trocknung kann zum Beispiel mit Hilfe eines geeigneten Trocknungsmittels in einer mit Stoffaustauschelementen bestückten Absorptionskolonne erfolgen. Ein geeignetes Trocknungsmittel kann, wie z.B. in DE 10 235 476 A beschrieben, neben Molsieben oder hygroskopischen Adsorbentien z.B. Schwefelsäure sein. Die Trocknung kann ein- oder mehrstufig erfolgen. Bevorzugt erfolgt die Trocknung zwei-stufig, indem das zu trocknende Chlor in einer ersten Stufe mit einer Schwefelsäure geringerer Konzentration, vorzugsweise 70 bis 80%, besonders bevorzugt 75 bis 80%, in Kontakt gebracht wird. In einer zweiten Stufe wird die Restfeuchte mittels einer höher konzentrierten Schwefelsäure von bevorzugt 88 bis 96%, besonders bevorzugt 92-96%, aus dem Chlor entfernt. Das auf diese Art getrocknete Chlor mit einer Restfeuchte von bevorzugt maximal 100 ppm, besonders bevorzugt maximal 20 ppm kann durch einen Tröpfchenabscheider geleitet werden, um gegebenenfalls darin noch enthaltene Schwefelsäuretröpfchen zu entfernen.

Die Kreislauffahrweise des erfindungsgemäßen Verfahrens erfordert es, neben dem gemäß Schritt (g) mittels Elektrolyse oder thermische Oxidation hergestellten Chlors für die Phosgenherstellung gemäß Schritt (a) eine weitere Teilmenge Chlor bereitzustellen, da Verluste an Chlor und Chlorwasserstoff in dem Chlor-Chlorwasserstoff-Kreislauf auftreten. Die Bereitstellung einer weiteren Teilmenge Chlor kann in Form von elementarem Chlor aus einer externen Quelle, beispielsweise der Elektrolyse einer wäss-rigen Natriumchloridlösung, stammen. Die auftretenden Verluste von Chlor und Chlorwasserstoff können aber auch dadurch ausgeglichen werden, dass eine Teilmenge Chlorwasserstoff aus einer externen Quelle bereitgestellt wird, z.B. aus einer Isocyanatproduktionsanlage (e.g. MDI, TDI), bei dem Chlorwasserstoff als gasförmiges Nebenprodukt anfällt. Eine Teilmenge Chlorwasserstoff in Form einer wässrigen Chlorwasserstofflösung aus einer externen Quelle, z.B. aus einem Produk-tionsverfahren, bei dem eine wässrige Chlorwasserstofflösung als Nebenprodukt anfällt, wird vorzugsweise als ca 30 Gew.%-ige Salzsäure einer Elektrolyse zugeführt. Eine Salzsäure geringe-rer Konzentration kann alternativ der Absorption von Chlorwasserstoff zugeführt werden.

Das Phosgen kann wieder in die Herstellung des Diarylcarbonat gemaß Schritt (b) eingesetzt werden.

Durch Umesterung des gemäß Schritt (b) hergestellten Diarylcarbonats mit wenigstens einem Bisphenol ergibt ein Oligo-/Polycarbonat und Monophenol, das wiederum in Schritt (b) eingesetzt werden kann.

Für das erfindungsgemäße Verfahren geeignete Bisphenole sind Dihydroxydiarylalkane der Formel (II),

HO-Z-OH (II)

worin Z ein divalenter organischer Rest mit 6 bis 30 Kohlenstoffatomen ist, der eine oder mehrere aromatische Gruppen enthält. Beispiele solcher Verbindungen, die in Schritt a) des erfindungsgemäßen Verfahrens eingesetzt werden können sind Dihydroxydiarylalkane wie Hydrochinon, Resorcin, Dihydroxydiphenyl, Bis-(hydroxyphenyl)-alkane, Bis(hydroxyphenyl)-cycloalkane, Bis-(hydroxyphenyl)-sulfide, Bis-(hydroxyphenyl)-ether, Bis-(hydroxyphenyl)-ketone, Bis-(hydroxyphenyl)-sulfone, Bis-(hydroxyphenyl)-sulfoxide, a,a'-Bis-(hydroxyphenyl)-diisopropylbenzole, so wie deren alkylierte, kernalkylierte und kernhalogenierte Verbindungen.

Bevorzugte Bisphenole sind 4,4'-Dihydroxydiphenyl, 2,2-Bis-(4-hydroxyphenyl)-1-phenylpropan, 1,1-Bis-(4-hydroxyphenyl)-phenylethan, 2,2-Bis-(4-hydroxyphenyl)-propan (Bisphenol A (BPA)), 2,4-Bis-(4-hydroxyphenyl)-2-methylbutan, 1,3-Bis-[2-(4-hydroxyphenyl)-2-propyl]-benzol (Bisphenol M), 2,2-Bis-(3-methyl-4-hydroxyphenyl)-propan, Bis-(3,5-dimethyl-4-hydroxyphenyl)-methan, 2,2-Bis-(3,5-dimethyl-4-hydroxyphenyl)-propan, Bis-(3,5-dimethyl-4-hydroxyphenyl)-sulfon, 2,4-Bis-(3,5-dimethyl-4-hydroxyphenyl)-2-methylbutan, 1,3-Bis-[2-(3,5-dimethyl-4-hydroxyphenyl)-2-propyl]-benzol, 1,1-Bis-(4-hydroxyphenyl)-cyclohexan und 1,1-Bis-(4-hydroxyphenyl)-3,3,5-trimethyl-cyclohexan (Bisphenol TMC).

Besonders bevorzugte Bisphenole sind 4,4'-Dihydroxydiphenyl, 1,1-Bis-(4-Hydroxyphenyl)-phenylethan, 2,2-Bis-(4-hydroxyphenyl)-propan (Bisphenol A (BPA)), 2,2-Bis(3,5-dimethyl-4-hydroxyphenyl)-propan, 1,1-Bis-(4-hydroxyphenyl)-cyclohexan und 1,1-Bis-(4-hydroxyphenyl)-3,3,5-trimethylcyclohexan (Bisphenol TMC).

Geeignete Bisphenole sind z.B. in den US 2 999 835 A, US 3 148 172 A, US 2 991 273 A, US 3 271 367 A, US 4 982 014 A und US 2 999 846 A in den deutschen Offenlegungsschriften DE 15 70 703 A, DE 20 63 050 A, DE 20 36 052 A, DE 22 11 956 A und DE 38 32 396 A, der französischen Patentschrift FR 1 561 518 A, in der Monographie von H. Schnell, Chemistry and Physics of Polycarbonates, Interscience Publishers, New York 1964, S. 28ff; S.102ff und von D.G. Legrand, J.T. Bendler, Handbook of Polycarbonate Science and Technology, Marcel Dekker New York 2000, S. 72ff. beschrieben.

Im Falle der erfindungsgemäßen Herstellung von Homopolycarbonaten wird nur ein Bisphenol eingesetzt, im Falle der erfindungsgemäßen Herstellung von Copolycarbonaten werden mehrere Bisphenole eingesetzt, wobei selbstverständlich die verwendeten Bisphenole, wie auch alle anderen der Synthese zugesetzten Chemikalien und Hilfsstoffe mit den aus ihrer eigenen Synthese, Handhabung und Lagerung stammenden Verunreinigungen kontaminiert sein können, obwohl es wünschenswert ist, mit möglichst sauberen Rohstoffen zu arbeiten.

Es sei hier betont, dass das erfindungsgemäße Verfahren praktisch für alle bekannten Bisphenole eingesetzt werden kann.

Die Polycarbonate können durch den Einsatz geringer Mengen Kettenabbrecher und Verzweiger bewusst und kontrolliert modifiziert werden. Geeignete Kettenabbrecher und Verzweiger sind literaturbekannt. Einige sind beispielsweise in der DE-A 38 33 953 beschrieben. Bevorzugt eingesetzte Kettenabbrecher sind Phenol oder Alkylphenole, insbesondere Phenol, p-tert.Butylphenol, iso-Octylphenol, Cumylphenol, deren Chlorkohlensäureester oder Säurechloride von Monocarbonsäuren bzw. Gemischen aus diesen Kettenabbrechern. Bevorzugte Kettenabbrecher sind Phenol, Cumyl-phenol, Isooctylphenol, para-tert.-Butylphenol.

Beispiele für als Verzweiger geeignete Verbindungen sind aromatische oder aliphatische Verbindungen mit mehr als drei, bevorzugt drei oder vier Hydroxygruppen. Besonders geeignete Beispiele mit drei oder mehr als drei phenolischen Hydroxylgruppen sind Phloroglucin, 4,6-Dimethyl-2,4,6-tri-(4-hydroxyphenyl)-hepten-2, 4,6-Dimethyl-2,4,6-tri-(4-hydroxyphenyl)-heptan, 1,3,5-Tri-(4-hydroxyphenyl)-benzol, 1,1,1-Tri-(4-hydroxyphenyl)-ethan, Tri-(4-hydroxyphenyl)-phenylmethan, 2,2-Bis-(4,4-bis-(4-hydroxyphenyl)-cyclohexyl]-propan, 2,4-Bis-(4-hydroxyphenyl-isopropyl)-phenol, Tetra-(4-hydroxyphenyl)-methan.

Beispiele für sonstige als Verzweiger geeignete trifunktionelle Verbindungen sind 2,4-Dihydroxybenzoesäure, Trimesinsäure, Cyanurchlorid und 3,3-Bis-(3-methyl-4-hydroxyphenyl)-2-oxo-2,3-dihydroindol.

Besonders bevorzugte Verzweiger sind 3,3-Bis-(3-methyl-4-hydroxyphenyl)-2-oxo-2,3-dihydroindol und 1,1,1 -Tri-(4-hydroxyphenyl)-ethan.

Die gegebenenfalls mitzuverwendenden 0,05 bis 2 Mol-%, bezogen auf eingesetzte Bisphenole, an Verzweigern, können mit den Bisphenolen zusammen eingesetzt werden.

Es ist darauf zu achten, dass die Reaktionskomponenten für den ersten Schritt, die Umesterung, also, die Bisphenole und die Diarylcarbonate frei von Alkali und Erdalkaliionen sind, wobei Mengen von kleiner 0,1 ppm an Alkali- und Erdalkaliionen toleriert werden können. Derart reine Bisphenole bzw. Diarylcarbonate sind erhältlich, indem man die Bisphenole bzw. Diarylcarbonate umkristallisiert, wäscht oder destilliert. Beim erfindungsgemäßen Verfahren soll der Gehalt an Alkali- und Erdalka-limetallionen sowohl im Bisphenol als auch im Diarylcarbonate einen Wert von < 0,1 ppm betragen.

Die Umesterungsreaktion des Bisphenols und des Diarylcarbonats in der Schmelze wird bevorzugt in zwei Stufen durchgeführt. In der ersten Stufe findet das Aufschmelzen des Bisphenols und des Diarylcarbonats bei Temperaturen von 80 - 250°C, bevorzugt 100 - 230°C, besonders bevorzugt 120 - 190°C unter normalem Druck in 0-5 Stunden, bevorzugt 0,25 - 3 Stunden statt. Nach Zugabe des Katalysators wird durch Anlegen von Vakuum (bis zu 2 mm Hg) und Erhöhung der Temperatur (auf bis zu 260°C) durch Abdestillieren des Monophenols das Oligocarbonat aus dem Bisphenol und dem Diarylcarbonat hergestellt. Das so hergestellte Oligocarbonat hat eine mittlere Gewichtsmol-masse M_{w} (ermittelt durch Messung der rel. Lösungsviskosität in Dichlormethan oder in Mischungen gleicher Gewichtsmengen Phenol / o-Dichlorbenzol, geeicht durch Lichtstreuung) im Bereich von 2000 bis 18 000 bevorzugt von 4 000 bis 15 000. Hierbei wird die Hauptmenge an Monophenol (80 %) aus dem Prozeß wiedergewonnen.

In der zweiten Stufe wird bei der Polykondensation durch weiteres Erhöhen der Temperatur auf 250 - 320°C, bevorzugt 270 - 295°C und einem Druck von < 2 mm Hg das Polycarbonat hergestellt. Hierbei wird der Rest an Monophenolen zurückgewonnen. Es können geringe Verluste an Monophenolen < 5 %, bevorzugt < 2 %, besonders bevorzugt < 1 % auftreten, verursacht durch Endgruppen im Polycarbonat und Restmonophenol im Polycarbonat. Diese Verluste müssen durch entsprechende Mengen an Monophenol für die Herstellung des Diarylcarbonats ausgeglichen werden.

Katalysatoren im Sinne des erfindungsgemäßen Verfahren für die Umesterung des Bisphenols und des Diarylcarbonats zum Polycarbonat sind alle anorganische oder organischen basischen Verbindungen beispielsweise Lithium-, Natrium-, Kalium-, Cäsium-, Calzium-, Barium-, Magnesium-, - hydroxide, -carbonate, -halogenide, -phenolate, -diphenolate, -fluoride, -acetate, -phosphate, - hydrogenphosphate, -boranate, Stickstoff- und Phosphorbasen wie beispielsweise Tetramethylammoniumhydroxid, Tetramethylammoniumacetat, Tetramethylammoniumfluorid, Tetramethylammoniumtetraphenylboranat, Tetraphenylphosphoniumfluorid, Tetra-phenylphosphoniumtetraphenylboranat, Dimethyldiphenylammoniumhydoxid, Tetraethylammoniumhydroxid, DBU, DBN oder Guanidinsysteme wie beispielsweise das 1,5,7-Triazabicyclo-[4,4,0]-dec-5-en, 7-Phenyl-1,5,7-triazabicyclo-[4,4,0]-dec-5-en, 7-Methyl-1,5,7-tria-zabicyclo-[4,4,0]-dec-5-en, 7,7'-Hexylidendi-1,5,7-triazabi-cyclo-[4,4,0]-dec-5-en, 7,7'-Decyliden-di-1,5,7-triazabicyclo-[4,4,0]-dec-5-en, 7,7'-Dodecyliden-di-1,5,7-tri-aza-bicyclo-[4,4,0]-dec-5-en oder Phosphazene wie beispielsweise die Phosphazen-Base P₁-t-Oct = tert.-Octyl-imino-tris-(dimethylamino)-phosphoran, Phosphazen-Base P₁-t-Butyl = tert.-Butyl-imino-tris-(dimethylamino)-phosphoran, BEMP = 2-tert.-Butylimino-2-diethylamino-1,3-dimethyl-perhydro-1,3,2-diaza-2-phosphoran.

Diese Katalysatoren werden in Mengen von 10⁻² bis 10⁻⁸ Mol, bezogen auf 1 Mol Bisphenol, eingesetzt.

Die Katalysatoren können auch in Kombination (zwei oder mehrere) miteinander eingesetzt werden.

Beim Einsatz von Alkali-/Erdalkali-Metallkatalysatoren kann es vorteilhaft sein, die Alkali-/Erdalkali-Metallkatalysatoren zu einem späteren Zeitpunkt (z.B. nach der Oligocarbonatsynthese bei der Polykondensation in der zweiten Stufe) zuzusetzen. Die Zugabe des Alkali-/ErdalkaliMetall-katalysators kann z.B. als Feststoff oder als Lösung in Wasser, Phenol, Oligocarbonat oder Poly-carbonat erfolgen.

Die Mitverwendung von Alkali- bzw. Erdalkali-Metallkatalysatoren widerspricht nicht der vorstehend erwähnten Forderung nach Reinheit der Reaktionspartner.

Die Reaktion des Bisphenols und des Diarylcarbonats zum Polycarbonat kann im Sinne des erfindungsgemäßen Verfahrens diskontinuierlich oder bevorzugt kontinuierlich durchgeführt werden, beispielsweise in Rührkesseln, Dünnschichtverdampfern, Fallfilmverdampfern, Rührkesselkaskaden, Extrudern, Knetern, einfachen Scheibenreaktoren und Hochviskosscheibenreaktoren.

Die aromatischen Polycarbonate des erfindungsgemaßen Verfahrens sollen mittlere Gewichtsmolmassen M_{w} von 18000 bis 80000 vorzugsweise 19000 bis 50000 haben, ermittelt durch Messung der rel. Lösungsviskosität in Dichlormethan oder in Mischungen gleicher Gewichtsmengen Phenol / o-Dichlorbenzol, geeicht durch Lichtstreuung.

Es ist vorteilhaft, die aus der Umesterung des Bisphenols und des Diarylcarbonats zum Polycarbonat abgespaltenen und isolierten Monophenole vor dem Einsatz in die Diarylcarbonat-synthese aufzureinigen. Die Rohmonophenole, die beim Umesterungsprozeß isoliert werden, können je nach Umesterungsbedingungen und Destillationsbedingungen u.a. mit Diarylcarbonaten, dem Bisphenol, Salicylsäure, Isopropenylphenol, Phenoxybenzoesäurephenylester, Xanthon, dem Hydroxymonoarylcarbonat verunreinigt sein. Die Reinigung kann nach den üblichen Reinigungs-verfahren, also z. B. Destillation oder Umkristallisation erfolgen. Die Reinheit der Monophenole liegt dann bei > 99 %, bevorzugt bei > 99,8 %, besonders bevorzugt bei > 99,95 %.

Der Vorteil des erfindungsgemäßen integrierten Verfahrens zur Herstellung von Diarylcarbonaten und Polycarbonaten unter thermischer oder elektrochemischer Oxidation des bei der Diarylcarbonatherstellung anfallenden Chlorwasserstoffs zur Rückgewinnung von Chlor für die Synthese von Phosgen ist die Wiederverwendung des Katalysators wodurch die Wirtschaftlichkeit des Diphenylcarbonatherstellungsverfahrens weiter erhöht als auch die Umweltbelastung durch Verringerung der Emissionen reduziert.

Die Herstellung von Diarylcarbonaten und Polycarbonaten im Verbund mit der elektrochemischen Oxidation von Salzsäure bietet durch die Erzeugung einer aufkonzentrierten Salzsäure von rund 30% aus einer Salzsäure von rund 17% in Schritt e) zusätzlich die Möglichkeit, bei Bedarf aufkonzentrierte Salzsäure für andere Anwendungen dem Kreislauf zu entnehmen. Eine mögliche Verwendung dieser aufkonzentrierten Salzsäure liegt im Nahrungsmittelbereich. Hierfür kann für die nach dem erfindungsgemäßen Verfahren hergestellte aufkonzentrierte Salzsäure z.B. durch absorptive Nachreinigung an einem Aktivkohlebett, wie sie aus dem Stand der Technik bekannt ist, eine für die Nahrungsmittelindustrie ausreichend hohe Reinheit erreicht werden.

Das erfindungsgemäße Verfahrens zur Herstellung von Diarylcarbonaten und Polycarbonaten kann gegebenenfalls mit anderen Einsatzmöglichkeiten des bei der Diarylcarbonatherstellung anfallenden Chlorwasserstoffs wie z. B. die Verwendung als Edukt für die Ethylendichloridherstellung bei der PVC-Produktion kombiniert werden.

Die Beispiele sollen das erfindungsgemäße Verfahren anhand der Herstellung von Diphenylcarbonat unter Wiederverwendung des Katalysators das Diphenylcarbonat-Herstellungsverfahren darstellen. Das bei der Polycarbonatherstellung anfallende Phenol wird in die Her-stellung von Diphenylcarbonat rückgeführt.
**Fig. 1** zeigt ein integriertes Verfahren zur Herstellung von Polycarbonat mit Rückführung des anfallenden Chlorwasserstoffs und des Phenols.

### Beispiele

Die Beispiele sollen das erfindungsgemäße Verfahren anhand der Rückführung der bei der Herstellung von Diphenylcarbonat eingesetzten Katalysatorfraktion, die wieder in das Diphenylcarbonat-Herstellungsverfahren (DPC-Verfahren) eingesetzt werden kann, unter Rückführung des bei der Herstellung von Polycarbonat gebildeten Phenols in die Herstellung von Diphenylcarbonat (Figur 1), darstellen.

### Beispiel 1

### Rückführung des Titan-haltigen Katalysators bei der Direktphosgenierung von Phenol

### 1a) Konti-Phosgenierung von Phenol in Gegenwart von TiCl₄ in einer Reaktivdestillationskolonne

Man dosiert von oben in eine auf 150°C thermostatisierte Reaktivdestillationsapparatur mit 20 Böden von je 43 Volumeteilen unter Normaldruck 187,3 Gew.-Teile/h aufgeschmolzenes Phenol und 0,42 Gew.-Teile/h TiCl₄ und gleichzeitig von unten 62,7 Gew.-Teile/h auf gleicher Temperatur erhitztes Phosgen in Gegenstrom.

Das am Fuß austretende Produkt, enthaltend Phenol, Chlorameisensäurephenylester, Diphenylcarbonat und Nebenprodukte (Gewichtsverhältnis 31,7/5,2/62,8/0,3) (Phosgenumsatz 83,5%, Phenolumsatz 64,8%, Selektivität 99,7%) wird nach Umsetzung des vorhandenen Chlorameisensäurephenylesters und Phenols zu Diphenylcarbonat in einem Verweilzeitreaktor isoliert und durch Entgasung in Abgas und Sumpf (Gewichtsverhältnis Phenol, Diphenylcarbonat und Nebenprodukte 29,2/70,4/0,4) aufgetrennt.

Nach Destillation erhält man 99,8%-iges Diphenylcarbonat.

Der Destillationsrückstand enthält 21,0% Titan

Der am Kopf der Reaktivdestillationsanlage austretende Chlorwasserstoff enthaltende Abgasstrom wird mit dem Abgas aus der Entgasung vereinigt und von geringen Mengen Phosgen und Phenol durch Ausfrieren befreit. Der Chlorwasserstoff kann gegebenenfalls nach einer weiteren Reinigung einer Elektrolyse oder einer thermischen Oxidation zugeführt werden.

### 1b) Batch-Phosgenierung von Phenol in Gegenwart des Titan-haltigen Destillationsrückstands aus der Konti-Phosgenierung von Phenol in Gegenwart von TiCl₄

In einer 250 ml Planschlifftopf mit Wellenbrecher, Rührer und Kühler legt man 141,2 g (1,5 Mol) Phenol in Gegenwart von 0,25 Mol-% Titan-haltigen Destillationsrückstands (bezogen auf Ti) aus 1a) vor und leitet während 2h 0.89 Mol/h Phosgen durch. Das Phosgen und HCl enthaltende Abgas wird über zwei Kohletürme mit Wasser geleitet.
Nach vollständiger Phosgenzugabe und noch zwei h Rühren bei 140°C werden Proben genommen und gaschromatographisch analysiert. Man erhält 23,3% Phenolumsatz mit einer Selektivität von 99,6% und einem Molverhältnis Diphenylcarbonat/Chlorameisensäurephenylester von 100 zu 0.

### 1c) Konti-Phosgenierung von Phenol in Gegenwart des Titan-haltigen Destillationsrückstands aus der Konti-Phosgenierung von Phenol in Gegenwart von TiCl₄

Man dosiert von oben in eine auf 151°C thermostatisierte Reaktivdestillationsapparatur mit 20 Böden von je 43 Volumeteilen unter Normaldruck 199,7 Gew.-Teile/h aufgeschmolzenes Phenol und 0,342 Gew.-Teile/h des Titan-haltigen Destillationsrückstands aus 1a) (0,072 Gew.-Teile/h Titan) und gleichzeitig von unten 50,1 Gew.-Teile/h auf gleicher Temperatur erhitztes Phosgen in Gegenstrom.

Das am Fuß austretende Produkt, enthaltend Phenol, Chlorameisensäurephenylester, Diphenylcarbonat und Nebenprodukte, entspricht 45,1% Phenolumsatz mit einer Selektivität von 99,8% und einem Molverhältnis Diphenylcarbonat/Chlorameisensäurephenylester von 86,9 zu 13,1. Es wird nach Umsetzung des vorhandenen Chlorameisensäurephenylesters und Phenols zu Diphenylcarbonat in einem Verweilzeitreaktor isoliert und durch Entgasung in Abgas und Sumpf aufgetrennt.

Nach Destillation erhält man 99,9%-iges Diphenylcarbonat.

### Beispiel 2

### Rückführung des Aluminium-haltigen Katalysators bei der Direktphosgenierung von Phenol

### 2a) Konti-Phosgenierung von Phenol in Gegenwart von AlCl₃ in einer Reaktivdestillationskolonne

Man dosiert von oben in eine auf 150°C thermostatisierte Reaktivdestillationsapparatur mit 20 Böden von je 43 Volumeteilen unter Normaldruck 399,0 Gew.-Teile/h aufgeschmolzenes Phenol und 0,61 Gew.-Teile/h AlCl₃ und gleichzeitig von unten 100,0 Gew.-Teile/h auf gleicher Temperatur erhitztes Phosgen in Gegenstrom.

Das am Fuß austretende Produkt, enthaltend Phenol, Chlorameisensäure phenylester, Diphenylcarbonat und Nebenprodukte (Gewichtsverhältnis 60,7/3,5/34,8/0,6) (Phosgenumsatz 71,6%, Phenolumsatz 35,4%, Selektivität 99%) wird nach Umsetzung des vorhandenen Chlorameisensäurephenylesters mit Phenol zu Diphenylcarbonat in einem Verweilzeitreaktor isoliert und durch Entgasung in Abgas und Sumpf (Gewichtsverhältnis Phenol, Diphenylcarbonat und Nebenprodukte 59,6/39,6/0,8) aufgetrennt.

Das Abgas wird mit dem am Kopf der Reaktivdestillationsanlage austretende Abgasstrom vereinigt und von geringen Mengen Phosgen und Phenol durch Ausfrieren befreit.

Nach Destillation bei 172°C/22 mbar wird 99,7%-iges Diphenylcarbonat erhalten.

Der Destillationsrückstand enthält 1,1 Gew.-% Aluminium.

### 2b) Batch-Phosgenierung von Phenol in Gegenwart des Aluminium-haltigen Destillationsrückstands aus der Konti-Phosgenierung von Phenol in Gegenwart von AlCl₃

In einer 250 ml Planschlifftopf mit Wellenbrecher, Rührer und Kühler legt man 141.2 g (1.5 Mol) Phenol in Gegenwart von 0.25 Mol-% Aluminium-haltigen Destillationsrückstands (bezogen auf A1) aus 2a) vor und leitet während 2h 0.89 Mol/h Phosgen durch. Das Phosgen und HCl enthaltende Abgas wird über zwei Kohletürme mit Wasser geleitet.
Nach vollständiger Phosgenzugabe und weiter zwei h Rühren bei 140°C werden Proben genommen und gaschromatographisch analysiert. Man erhält 24,1% Phenolumsatz mit einer Selektivität von 97,9% und einem Molverhältnis Diphenylcarbonat/Chlorameisensäurephenylester von 99,1 zu 0,1.

### Mehrmalige Rückführung des Titan-haltigen Katalysatorfraktion bei der Direktphosgenierung von Phenol

### Beispiel 3

### Batch-Phosgenierung von Phenol in Gegenwart von 0,25 Mol-% Titan-haltigem Katalysator

### 3a) Batch-Phosgenierung von Phenol in Gegenwart von 0.25 Mol-% TiCl₄

In einer 250 ml Planschlifftopf mit Wellenbrecher, Rührer und Kühler legt man 141.2 g (1.5 Mol) Phenol in Gegenwart von 0.25 Mol-% TiCl₄ vor und leitet während 2h 0.89 Mol/h Phosgen durch. Das Phosgen und HCl enthaltende Abgas wird über zwei Kohletürme mit Wasser geleitet.

Nach vollständiger Phosgenzugabe und noch zwei h Rühren bei 140°C werden Proben genommen und gaschromatographisch analysiert. Man erhält 75,8% Phenolumsatz mit einer Selektivität von 99,3%und einem Molverhältnis Diphenylcarbonat/Chlorameisensäurephenylester von 100 zu 0. Die Reaktionsmischung wird destillativ aufgetrennt und der Titangehalt des Rückstands bestimmt.

### 3b) Batch-Phosgenierung von Phenol in Gegenwart des Titan-haltigen Destillationsrückstands aus 3a) (1. Katalysatorrückführung)

Nach dem in 3a) beschriebenen Verfahren legt man Phenol in Gegenwart des Titan-haltigen Destillationsrückstands aus 3a) vor und leitet während 2h Phosgen durch, wobei Phenol und Phosgenmenge 0,25 Mol-% Titan entsprechen. Nach vollständiger Phosgenzugabe und noch zwei h Rühren bei 140°C werden Proben genommen und gaschromatographisch analysiert.
Man erhält 76,8% Phenolumsatz mit einer Selektivität von 98,9% und einem Molverhältnis Diphenylcarbonat/Chlorameisensäurephenylester von 100 zu 0.

### 3c) Batch-Phosgenierung von Phenol in Gegenwart des Titan-haltigen Destillationsrückstands aus 3b) (2. Katalysatorrückführung)

Nach dem in 3b) beschriebenen Verfahren legt man Phenol in Gegenwart des Titan-haltigen Destillationsrückstands aus 3b) vor und leitet während 2h Phosgen durch, wobei Phenol und Phosgenmenge wieder 0,25 Mol-% Titan entsprechen.
Man erhält 75,4% Phenolumsatz mit einer Selektivität von 98,6% und einem Molverhältnis Diphenylcarbonat/Chlorameisensäurephenylester von 100 zu 0.

### 3d) Batch-Phosgenierung von Phenol in Gegenwart des Titan-haltigen Destillationsrückstands aus 3c) (3. Katalysatorrückführung)

Nach dem in 3b) beschriebenen Verfahren legt man Phenol in Gegenwart des Titan-haltigen Destillationsrückstands aus 3c) vor und leitet während 2h Phosgen durch, wobei Phenol und Phosgenmenge wieder 0,25 Mol-% Titan entsprechen.
Man erhält 85,8% Phenolumsatz mit einer Selektivität von 98,4% und einem Molverhältnis Diphenylcarbonat/Chlorameisensäurephenylester von 100 zu 0.

### Beispiel 4

### Batch-Phosgenierung von Phenol in Gegenwart von 0,125 Mol-% Titan-haltigem Katalysator

### 4a) Batch-Phosgenierung von Phenol in Gegenwart von 0,125 Mol-% TiCl₄

In einer 250 ml Planschlifftopf mit Wellenbrecher, Rührer und Kühler legt man 141.2 g (1.5 Mol) Phenol in Gegenwart von 0,125 Mol-% TiCl₄ vor und leitet während 2h 0.89 Mol/h Phosgen durch. Das Phosgen und HCl enthaltende Abgas wird über zwei Kohletürme mit Wasser geleitet.
Nach vollständiger Phosgenzugabe und noch zwei h Rühren bei 140°C werden Proben genommen und gaschromatographisch analysiert. Man erhält eine Selektivität von 99,5% und einem Molverhältnis Diphenylcarbonat/Chlorameisensäurephenylester von 100 zu 0.
Die Reaktionsmischung wird destillativ aufgetrennt und der Titangehalt des Rückstands bestimmt.

### 4b) Batch-Phosgenierung von Phenol in Gegenwart von 0,125 Mol-% Titan-haltigem Destillationsrückstands

Nach dem oben beschriebenen Verfahren legt man Phenol in Gegenwart des Titan-haltigen Destillationsrückstands aus 4a) vor und leitet während 2h Phosgen durch, wobei Phenol und Phosgenmenge wieder 0,125 Mol-% Titan entsprechen.
Nach Aufarbeitung und fünffacher Wiedereinsatz des jeweiligen Katalysatorrückstands erhält man eine Selektivität von 98,5%.

### Beispiel 5

### Batch-Phosgenierung von Phenol in Gegenwart von 0,0625 Mol-% Titan-haltigem Katalysator

### 5a) Batch-Phosgenierung von Phenol in Gegenwart von 0,0625 Mol-% TiCl₄

In einer 250 ml Planschlifftopf mit Wellenbrecher, Rührer und Kühler legt man 141.2 g (1.5 Mol) Phenol in Gegenwart von 0,0625 Mol-% TiCl₄ vor und leitet während 2h 0.89 Mol/h Phosgen durch. Das Phosgen und HCl enthaltende Abgas wird über zwei Kohletürme mit Wasser geleitet. Nach vollständiger Phosgenzugabe und noch zwei h Rühren bei 140°C werden Proben genommen und gaschromatographisch analysiert. Man erhält einerSelektivität von 99,3%.
Die Reaktionsmischung wird destillativ aufgetrennt und der Titangehalt des Rückstands bestimmt.

### 5b) Batch-Phosgenierung von Phenol in Gegenwart von 0,0625 Mol-% Titan-haltigem Destillationsrückstands.

Nach dem oben beschriebenen Verfahren legt man Phenol in Gegenwart des Titan-haltigen Destillationsrückstands aus 5a) vor und leitet während 2h Phosgen durch, wobei Phenol und Phosgenmenge wieder 0,0625 Mol-% Titan entsprechen.

Nach Aufarbeitung und achtfacher Wiedereinsatz des jeweiligen Katalysatorrückstands erhält man eine Selektivität von 98,5%.

### Beispiel 6

### Reinigung des Destillationsrückstands durch Umwandlung von Ti(OC₆H₅)₄ in TiCl₄

### Reaktion von Ti(OC₆H₅)₄ mit Phosgen

Einleitung von 0,253 bzw. 1,012 Mol/h Phosgen in eine Blasensäuleapparatur aus zwei nacheinander geschalteten Blasensäulen, wobei die 1. Blasensäule (220°C) mit Diphenylcarbonat und Ti(OC₆H₅)₄ (177 mg Ti) bestückt worden ist, während die 2. Blasensäule (120°C) nur Phenol enthält. Nach Umwandlung des Ti(OC₆H₅)₄ in TiCl₄ und Phenol in der 1. Blasensäule und Überdestillieren des TiCl₄ in die 2. Blasensäule findet dort Direktphosgenierung von Phenol zu Chlorameisensäurephenylester und Diphenylcarbonat statt.

Nach 5h Phosgeneinleitung (0,253 Mol/h) enthält der 1. Blasensäule Phenol, Chlorameisensäurephenylester und Diphenylcarbonat im Gewichtsverhältnis 0,1/14,6/84,7 (56 mg Ti), während der 2. Blasensäule Phenol, Chlorameisensäurephenylester und Diphenylcarbonat im Gewichtsverhältnis 54,4/6,8/38,6 (76 mg Ti) enthält.

Bei 5h Einleitung von 1,012 Mol/h Phosgen nach oben beschriebenen Verfahren enthält der 1. Blasensäule Phenol, Chlorameisensäurephenylester und Diphenylcarbonat im Gewichtsverhältnis 0,1/2,3/96,9 (31 mg Ti), während der 2. Blasensäule Phenol, Chlorameisensäurephenylester und Diphenylcarbonat im Gewichtsverhältnis 2,8/11,5/85,5 (77 mg Ti) enthält.

### Beispiel 7

### Herstellung von Polycarbonat

Aus einer Vorlage werden 8.600 Gew.-Teile/h Schmelzegemisch, bestehend aus 4.425 Gew.-Teilen/h Diphenylcarbonat, hergestellt wie beschrieben in 1a) oder 1b), und 4.175 Gew.-Teilen/h Bisphenol A, unter Zusetzen von 0,52 Gew.-Teilen/h des Phenoladdukts von Tetraphenylphosphoniumphenolat mit 65,5 % Tetraphenylphosphoniumphenolat/h gelöst in 4,5 Gew.-Teile/h Phenol/h durch einen Wärme-tauscher gepumpt, auf 190°C erwärmt und durch eine Verweilkolonne bei 12 bar und 190°C geführt. Die mittlere Verweilzeit beträgt 50 Minuten.

Die Schmelze wird dann über ein Entspannungsventil in einen unter 200 mbar stehenden Abscheider geleitet. Die abfließende Schmelze wird in einem, ebenfalls unter 200 mbar stehenden, Fallfilmver-dampfer wieder auf 189°C erwärmt und in einer Vorlage aufgefangen. Nach einer Verweilzeit von 20 Minuten wird die Schmelze in die nächsten drei, gleichartig aufgebauten Stufen gepumpt. Die Bedingungen in der 2. / 3. / 4. Stufe sind 100 / 74 / 40 mbar, 218 / 251 / 276°C und 20 / 10 / 10 Minuten. Das entstandene Oligomere hat eine relative Viskosität von 1,09. Alle Brüden werden über Druckregelungen in eine unter Vakuum stehende Kolonne geführt und als Kondensate abgeleitet.

Danach wird das Oligomer in einem sich anschliessenden Korbreaktor bei 278°C und 3,0 mbar bei einer Verweilzeit von 45 Minuten zu einem höhermolekularen Produkt aufkondensiert. Die relative Viskosität beträgt 1,195. Die Brüden werden kondensiert.

Vom Schmelzestrom, der in einen weiteren Korbreaktor geleitet wird, wird mittels einer Zahnradpumpe ein Teilstrom von 150 Gew.-Teilen/h Schmelze abgezweigt, mit 0,185 Gew.-Teilen/h einer 5 %-igen wässrigen Phosphorsäure versetzt, über einen statischen Mischer mit einem Länge-zu-Durchmesser-Verhältnis von 20 gerührt und wieder in den Hautschmelzestrom zurückgeleitet. Direkt nach dem Zusammentreffen wird die Phosphorsäure im gesamten Schmelzestrom mittels eines weiteren statischen Mischers homogen verteilt.

Die so behandelte Schmelze wird in einem weiteren Korbreaktor bei 284°C, 0,7 mbar und bei einer mittleren Verweilzeit von 130 Minuten weiter den Prozessbedingungen ausgesetzt, ausgetragen und granuliert.

Die Brüden werden in der Vakuumanlage und dahinter kondensiert.

Das erhaltene Polycarbonat hat folgende Kennzahlen: relative Viskosität 1,201 / phenolische OH 255 [ppm] / DPC 71 [ppm] / BPA 6 [ppm] / Phenol 56 [ppm].

Das abdestillierte Phenol kann wieder in die Diphenylcarbonatherstellung gemäß Schritt (b), wie beschrieben in den Beispielen 1 - 5, rückgeführt werden.

## Patentansprüche

1. Verfahren zur Herstellung von Diarylcarbonat umfassend folgende Schritte:
(a) Herstellung von Phosgen durch Umsetzung von Chlor mit Kohlenmonoxid,
(b) Umsetzung des gemäß Schritt a) gebildeten Phosgens mit wenigstens einem Monophenol in Gegenwart eines Metall-Katalysators, und gegebenenfalls organischem Lösungsmittel unter Bildung wenigstens eines Diarylcarbonats und von Chlorwasserstoff,
(c) Abtrennung und Aufarbeitung des in Schritt b) gebildeten Diarylcarbonats
(d) Rückführung der gemäß Schritt (c) zurückbleibenden schwerflüchtigen Metall enthaltenden Katalysatorfraktion in die Herstellung des Diarylcarbonats gemäß Schritt (b),
(e) Abtrennung und gegebenenfalls Reinigung des gemäß Schritt (b) gebildeten Chlorwasserstoffs
(f) elektrochemische oder thermische Oxidation wenigstens eines Teils des Chlorwasserstoffs aus (e) zu Chlor
(g) Rückführung wenigstens eines Teils des gemäß Schritt (f) hergestellten Chlors in die Herstellung von Phosgen gemäß Schritt (a),
wobei gegebenenfalls die Metall enthaltende Katalysatorfraktion vor der Rückführung mit Chlorierungsmitteln wie beispielsweise Phosgen, Chlorameisensäurephenylester oder Chlorwasserstoff umgesetzt und gereinigt wird.

2. Verfahren nach Anspruch 1, **dadurch gekennzeichnet, dass** die bei der Umsetzung mit der Metall enthaltenden Katalysatorfraktion eingesetzte Substanz Phosgen aus Schritt (a) ist.

3. Verfahren nach Anspruch 1, **dadurch gekennzeichnet, dass** die bei der Umsetzung mit der Metall enthaltenden Katalysatorfraktion eingesetzte Substanz Chlorwasserstoff aus Schritt (e) ist.

4. Verfahren nach Anspruch 1 bis 3, **dadurch gekennzeichnet, dass** die Metall enthaltenden Katalysatorfraktion Titan oder Aluminium enthält.

5. Verfahren gemäß einem der Ansprüche 1 bis 4, **dadurch gekennzeichnet, dass**, Chlor gemäß Schritt (a) zu Phosgen umgesetzt und das so erhaltene Phosgen zur Umsetzung mit Phenol zu Diphenylcarbonat gemäß Schritt (b) eingesetzt wird.

6. Verfahren gemäß einem der Ansprüche 1 bis 5, **dadurch gekennzeichnet, dass** das Diarylcarbonat mit einem Bisphenol zum Oligo-/Polycarbonat und einem Monophenol umgesetzt und das so erhaltene Monophenol zur Umsetzung mit Phosgen zu Diphenylcarbonat gemäß Schritt (b) eingesetzt wird.

7. Verfahren gemäß einem der Ansprüche 1 bis 6, **dadurch gekennzeichnet, dass** es sich beim hergestellten Diarylcarbonat um Diphenylcarbonat handelt.
